(19) 

Europäisches Patentamt
European Patent Office
Office européen des brevets

(11) **EP 4 144 803 B1**

(12) **EUROPEAN PATENT SPECIFICATION**

(45) Date of publication and mention
of the grant of the patent:
**25.12.2024 Bulletin 2024/52**

(21) Application number: **21797475.7**

(22) Date of filing: **23.04.2021**

(51) International Patent Classification (IPC):
**C08L 101/14** (2006.01)   **A61F 13/15** (2006.01)
**A61F 13/53** (2006.01)   **C08K 9/04** (2006.01)

(52) Cooperative Patent Classification (CPC):
(C-Sets available)
**A61F 13/15203; A61F 13/53; A61F 13/8405;**
**B01J 20/22; B01J 20/267; B01J 20/28004;**
**B01J 20/28011; C08K 9/04; C08L 101/14;**
A61F 2013/530481; A61F 2013/8408;
B01J 2220/68; C08J 3/12; C08K 3/34; C08K 3/36;

(Cont.)

(86) International application number:
**PCT/JP2021/016525**

(87) International publication number:
**WO 2021/220982 (04.11.2021 Gazette 2021/44)**

(54) **WATER ABSORBENT RESIN COMPOSITION, ABSORBENT, AND ABSORBENT ARTICLE**

WASSERABSORBIERENDE HARZZUSAMMENSETZUNG, ABSORPTIONSMITTEL UND
SAUGFÄHIGER ARTIKEL

COMPOSITION DE RÉSINE ABSORBANT L'EAU, ABSORBANT ET ARTICLE ABSORBANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.04.2020 JP 2020078117**

(43) Date of publication of application:
**08.03.2023 Bulletin 2023/10**

(73) Proprietor: **Sumitomo Seika Chemicals Co., Ltd.**
**Kako-gun, Hyogo 675-0145 (JP)**

(72) Inventor: **MATSUOKA, Mutsumi**
**Himeji-shi, Hyogo 672-8076 (JP)**

(74) Representative: **Mewburn Ellis LLP**
**Aurora Building**
**Counterslip**
**Bristol BS1 6BX (GB)**

(56) References cited:
| | |
|---|---|
| EP-A1- 3 604 366 | WO-A1-2007/074816 |
| WO-A1-2016/103808 | WO-A1-2018/062531 |
| WO-A1-2018/180864 | JP-A- 2007 054 328 |
| JP-A- 2007 054 328 | JP-A- 2016 214 557 |
| JP-A- 2017 176 757 | JP-A- 2017 197 884 |
| JP-A- 2017 197 884 | JP-A- 2018 069 140 |
| JP-A- 2018 069 140 | JP-A- 2020 050 751 |
| US-A1- 2011 068 300 | US-A1- 2018 221 528 |
| US-A1- 2020 030 482 | |

**(Cont. next page)**

EP 4 144 803 B1

(52) Cooperative Patent Classification (CPC): (Cont.)
C08K 5/25; C08K 2201/005

C-Sets
**C08K 9/04, C08L 101/14**

**Description**

TECHNICAL FIELD

The present invention relates to a water-absorbing resin composition, an absorber, and an absorbent article, and more particularly to a water-absorbing resin composition constituting an absorber suitably used for hygienic materials such as disposable diapers, sanitary napkins, and incontinence pads, and an absorbent article using the absorber.

BACKGROUND ART

In recent years, water-absorbing resins have been widely used in the field of hygienic materials such as disposable diapers, sanitary napkins, and incontinence pads.

As such a water-absorbing resin, a crosslinked product of a polymer of a water-soluble ethylenically unsaturated monomer, more specifically, a crosslinked product of a polymer of a partially neutralized polyacrylic acid has excellent water absorbing capacity, and acrylic acid as a raw material thereof is easily industrially available, so that the crosslinked product of the polymer can be produced at a constant quality and at a low cost. Also, the crosslinked product of the polymer has many advantages such as being less likely to cause decomposition or deterioration, and thus it is considered to be a preferable water-absorbing resin.

On the other hand, an absorbent article such as a disposable diaper, a sanitary napkin, or an incontinence pad mainly includes an absorber that is disposed in a central portion and absorbs and holds body fluids such as urine and menstrual blood excreted from the body, a liquid-permeable surface sheet (top sheet) disposed on a side coming in contact with the body, and a liquid-impermeable back sheet (back sheet) disposed on a side opposite to the side coming in contact with the body. The absorber is usually composed of hydrophilic fibers such as pulp and a water-absorbing resin.

When such an absorber is used for, for example, a hygienic material, an unpleasant odor of acetaldehyde and the like may be generated from the absorber that has absorbed body fluids, particularly urine, blood, sweat, and the like.

As a method for suppressing such an unpleasant odor, for example, a method in which an organic amine compound such as a hydrazide compound is added to a water-absorbing resin as an adsorbent for an aldehyde compound is known (see JP 2001-323155 A).

JP 2007-054328 A concerns a deodorant comprising a metal complex of hydrazide compound. US 2018/0221528 A1 concerns a deodorant composition comprising an amorphous aluminum silicate, a hydrazide compound, and a crystalline zinc oxide in which at least two peaks among three peaks detected within a diffraction angle range from 30 to 38 degrees when the crystalline zinc oxide is subjected to X-ray powder diffraction measurement have a half width of 0.4 to 1.2 degrees is an X-ray diffraction chart, wherein a content of the crystalline zinc oxide is in a range from 100 to 500 parts by mass based on 100 parts by mass of the hydrazide compound, and wherein a content of the amorphous aluminum silicate is in a range from 100 to 750 parts by mass based on 100 parts by mass of the hydrazide compound. EP 3604366 A1 concerns a water-absorbent resin comprising a polymer of a water-soluble ethylenically unsaturated monomer. JP 2018-069140 A concerns an odor absorbent containing an acid hydrazide compound and silica, in which the silica has silanol group density of a surface of $5.0/nm^2$ or less. JP 2017-197884 A concerns a fiber cloth for sanitary products to which a deodorant antibacterial agent containing a composite of a metal salt and silicate, a porous substance, an amine compound, and a surfactant is solidly attached. US 2011/0068300 A1 concerns a water-absorbing resin compound comprising a water-absorbing resin, an antibacterial agent having a porous material incorporating an antibacterial metal, and a metal chelating agent. JP 2017-176757 A concerns a deodorant building material applied as a wall material or the like to the inside of a room, wherein a deodorant element-including layer is formed on the surface facing the room inside, and wherein an adsorption deodorant element deodorizing an odor component by adsorption, a chemical reaction deodorant element deodorizing by chemical reaction and a photocatalyst decomposing the odor component are provided in an exposed state in the deodorant element-including layer. WO 2007/074816 A1 concerns a purifier composition for aldehyde-containing air comprising as the active ingredient at least one kind of porous inorganic particles selected from silica gel particles and alumina particles which particles carry at least one hydrazide in an amount of 5 to 70 parts by weight per 100 parts of the particles with the void volumes of the porous inorganic particles ranging from 0.1 to 2.0ml/g. US 2020/0030482 A1 concerns a water absorbent resin composition comprising a water absorbent resin and a hydroxamic acid or a salt thereof. JP 2020-050751 A concerns a crosslinked polyolefin resin foam by foaming a foamable composition containing a polyolefin resin and a deodorant, wherein the foamable composition contains the deodorant of 0.2 to 6.5 parts by mass based on 100 parts by mass of the polyolefin resin, the deodorant contains a porous particle and specific hydrazide.

SUMMARY OF THE INVENTION

PROBLEMS TO BE SOLVED BY THE INVENTION

However, as a result of studies by the present inventors, it has become clear that, although the effect of suppressing odor can be exerted by adding the hydrazide compound to the water-absorbing resin, the water-absorbing resin containing the hydrazide compound is colored over time when stored in a specific environment for a certain period of time or longer. When the water-absorbing resin is colored yellow or brown, the commercial value of the absorber and the absorbent article containing the water-absorbing resin may be impaired.

It is a main object of the present invention to provide a water-absorbing resin composition that suppresses an acetaldehyde odor and suppresses coloring over time.

MEANS FOR SOLVING THE PROBLEM

The present inventors have conducted intensive studies in order to solve the above problems. As a result, the present inventors have found that by using a supported hydrazide compound in which a hydrazide compound is supported, together with a water-absorbing resin, mainly acetaldehyde is adsorbed, whereby its odor is suppressed, and coloring over time is suppressed.

The present invention has been completed through further intensive studies based on such findings.

That is, the present invention provides an invention having the following configuration.

Item 1. A water-absorbing resin composition including a supported hydrazide compound and a water-absorbing resin, wherein the water-absorbing resin composition has a yellow index of 10 or less when 2.0 g of the water-absorbing resin composition is held in an environment at a temperature of 70°C and a relative humidity of 90% for 7 days, and an acetaldehyde concentration of 15 ppm or less is measured when the water-absorbing resin composition is tested according to the acetaldehyde odor suppression test as described in EXAMPLES.

Item 2. The water-absorbing resin composition according to Item 1, wherein the water-absorbing resin composition is in particulate form, and the supported hydrazide compound is present on at least one of a surface and an inside of the water-absorbing resin composition.

Item 3. The water-absorbing resin composition according to Item 1 or 2, wherein a support of the supported hydrazide compound is at least one of silicic acid and a silicate.

Item 4. The water-absorbing resin composition according to any one of Items 1 to 3, wherein a content of the supported hydrazide compound is 0.001 to 10 mass%.

Item 5. The water-absorbing resin composition according to any one of Items 1 to 4, wherein a content of a hydrazide compound in the supported hydrazide compound is 0.1 to 30 mass%.

Item 6. The water-absorbing resin composition according to any one of Items 1 to 5, wherein the supported hydrazide compound has a median particle diameter of 0.01 to 100 $\mu$m.

Item 7. An absorber including the water-absorbing resin composition according to any one of Items 1 to 6.

Item 8. An absorbent article including the absorber according to Item 7 held between a liquid-permeable sheet and a liquid-impermeable sheet.

ADVANTAGES OF THE INVENTION

According to the present invention, it is possible to provide a water-absorbing resin composition that suppresses an acetaldehyde odor and suppresses coloring over time. Furthermore, according to the present invention, it is also possible to provide an absorbent article and an absorber using the water-absorbing resin composition.

Conventionally, it is known that a hydrazide compound exhibits an effect on acetaldehyde in a state where a liquid such as water is provided. Thus, it is presumed that when the hydrazide compound is added to the water-absorbing resin, the water-absorbing resin draws a liquid, so that the hydrazide compound exerts an effect on acetaldehyde. On the other hand, in the water-absorbing resin composition of the present invention, the hydrazide compound is contained as a supported hydrazide compound in the water-absorbing resin composition, and the effect on acetaldehyde can be exhibited even if the water-absorbing resin composition is not provided with a liquid. Thus, for example, when the water-absorbing resin composition of the present invention is used in an absorbent article, the effect of suppressing the acetaldehyde odor can also be exhibited in a portion of the water-absorbing resin composition that does not absorb liquid.

EMBODIMENTS OF THE INVENTION

1. Water-absorbing resin composition

The water-absorbing resin composition of the present invention contains a supported hydrazide compound and a water-absorbing resin, has a yellow index of 10 or less when 2.0 g of the water-absorbing resin composition is held in an environment at a temperature of 70°C and a relative humidity of 90% for 7 days, and an acetaldehyde concentration of 15 ppm or less is measured when the water-absorbing resin composition is tested according to the acetaldehyde odor suppression test as described in EXAMPLES. The water-absorbing resin composition of the present invention suppresses an acetaldehyde odor, and suppresses coloring over time by having this configuration. Hereinafter, the water-absorbing resin composition of the present invention will be described in detail.

The water-absorbing resin composition of the present invention has a yellow index of 10 or less when 2.0 g of the water-absorbing resin composition is held in an environment at a temperature of 70°C and a relative humidity of 90% for 7 days, and from the viewpoint of more suitably exerting the effect of the present invention, the yellow index is preferably 9.8 or less, and more preferably 9.5 or less. The initial yellow index of the water-absorbing resin composition (that is, the yellow index of 2.0 g of the water-absorbing resin composition before being held in the above environment) is preferably 8.0 or less, more preferably 7.5 or less, and even more preferably 7.0 or less. The lower limit of these yellow indexes is 0. These yellow indexes are values measured by the methods described in EXAMPLES.

Also, an acetaldehyde concentration of 15 ppm or less is measured when the water-absorbing resin composition is tested according to the acetaldehyde odor suppression test as described in EXAMPLES, and from the viewpoint of more suitably exerting the effect of the present invention, the acetaldehyde concentration is preferably 10 ppm or less, and more preferably 5 ppm or less. The lower limit of the acetaldehyde concentration is 0 ppm.

(Supported hydrazide compound)

The supported hydrazide compound contained in the water-absorbing resin composition of the present invention is one in which a hydrazide compound is supported on a support. From the viewpoint of more suitably exerting the effect of the present invention, examples of the hydrazide compound include monohydrazide compounds such as formohydrazide, acetohydrazide, propionic acid hydrazide, 4-methylbenzohydrazide, biphenyl-4-carboxylic acid hydrazide, 2-bromobenzohydrazide, 3-bromobenzohydrazide, 4-bromobenzohydrazide, 2-chlorobenzohydrazide, 3-chlorobenzohydrazide, 4-chlorobenzohydrazide, 3,4-dichlorobenzohydrazide, 2,4-dichlorobenzohydrazide, 2,4-dihydroxybenzohydrazide, 3-hydroxybenzohydrazide, 4-hydroxybenzohydrazide, isobutyric acid hydrazide, 3-methoxybenzohydrazide, 4-methoxybenzohydrazide, methylmaleic acid hydrazide, 1-naphthohydrazide, nicotinic acid hydrazide, 3-nitrophthalic acid hydrazide, 4-nitrophthalic acid hydrazide, n-octanohydrazide, palmitic acid hydrazide, 2-phenoxybenzohydrazide, phenylacetic acid hydrazide, 2-pyridinecarboxylic acid hydrazide, stearic acid hydrazide, 2-thiophenecarboxylic acid hydrazide, and L-tyrosine hydrazide; dihydrazide compounds such as malonic acid dihydrazide, succinic acid dihydrazide, adipic acid dihydrazide, carbohydrazide, isophthalic acid dihydrazide, phthalic acid dihydrazide, terephthalic acid dihydrazide, azelaic acid dihydrazide, sebacic acid dihydrazide, oxalic acid dihydrazide, dodecanedioic acid dihydrazide, oxalyl dihydrazide, and adipodihydrazide; and furthermore, polyvalent hydrazide compounds having tri, tetra, or more hydrazide groups. The hydrazide compound is preferably a dihydrazide compound, more preferably malonic acid dihydrazide, succinic acid dihydrazide, adipic acid dihydrazide, carbohydrazide, isophthalic acid dihydrazide, phthalic acid dihydrazide, terephthalic acid dihydrazide, azelaic acid dihydrazide, sebacic acid dihydrazide, oxalic acid dihydrazide, dodecanedioic acid dihydrazide, oxalyl dihydrazide, or the like, and even more preferably malonic acid dihydrazide. The hydrazide compound supported on the support may be one or two or more types.

From the viewpoint of more suitably exerting the effect of the present invention, the support for supporting the hydrazide compound is preferably a silicate or a silicic acid, and more preferably a silicate. The silicate is preferably a layered silicate mineral (phyllosilicate mineral). Furthermore, the layered silicate mineral is preferably kaolin (for example, lizardite ($Mg_3Si_2O_5(OH)_4$), kaolinite ($Al_2Si_2O_5(OH)_4$, berthierine ($Fe_{2.5}Al_{0.5}$)[$Si_{1.5}Al_{0.5}O_5(OH)_4$], or the like), a mica clay mineral (for example, a fluorine-containing mineral such as fluorphlogopite ($KMg_3(AlSi_3)O_{10}F_2$), phlogopite ($KMg_3(AlSi_3)O_{10}(F,OH)_2$), polylithionite ($KLi_2AlSi_4O_{10}(F,OH)_2$), or eastonite ($KMg_2Al(Al_2Si_2)O_{10}(F,OH)_2$)), smectite (for example, montmorillonite ($Ca/2,Na)_{0.3}(Mg,Fe^{2+})_3(Si,Al)_4O_{10}(OH)_2/4H_2O$ or the like)), a mixed layer mineral, a serpentine mineral (for example, serpentine ($Mg_3Si_2O_5(OH)_4$), talc (($Mg_3Si_4O_{10}(OH)_2$)), chlorite (for example, clinochlore (($Mg,Fe^{2+})_5Al(Si_3Al)O_{10}(OH)_8$, shamosite (($Fe^{2+},Mg,Fe^{3+})5Al(Si_3Al)O_{10}(OH)_8$), or the like), vermiculite, or the like. The support for supporting the hydrazide compound may be one or two or more types.

Preferred specific examples of the supported hydrazide compound include silicates on which malonic acid dihydrazide is supported (particularly, a layered silicate mineral on which malonic acid dihydrazide is supported). The supported hydrazide compound can be prepared by a known method. A commercially available product satisfying the requirements described in the present invention can also be used.

From the viewpoint of more suitably exerting the effect of the present invention, the content of the hydrazide compound in the supported hydrazide compound is preferably 0.1 to 30 mass%, more preferably 0.5 to 20 mass%, and even more preferably 1.0 to 10 mass%.

The median particle diameter of the supported hydrazide compound is preferably 0.01 to 100 $\mu$m, more preferably 0.1 to 50 $\mu$m, and even more preferably 1.0 to 10 $\mu$m. The median particle diameter of the supported hydrazide compound is a value measured according to the laser diffraction methods (JIS Z 8825: 2013).

In the water-absorbing resin composition of the present invention, the content of the supported hydrazide compound is preferably 0.001 to 10 mass%, more preferably 0.005 to 5 mass%, and even more preferably 0.05 to 2 mass%.

The water-absorbing resin composition of the present invention is preferably in particulate form. The supported hydrazide compound is preferably present on at least one of the surface and the inside of the particulate water-absorbing resin, and more preferably present on the surface of the particulate water-absorbing resin. For example, by mixing the particulate water-absorbing resin and the supported hydrazide compound in a solid phase state, the supported hydrazide compound can be present on the surface of the particulate water-absorbing resin composition to such an extent that the effect of the present invention can be exhibited. The water-absorbing resin composition of the present invention may be prepared by mixing the supported hydrazide compound in a state of being dissolved or dispersed in a liquid medium such as an aqueous liquid, with the particulate water-absorbing resin. A supported hydrazide compound may be contained inside the particulate water-absorbing resin.

(Water-absorbing resin)

The water-absorbing resin contained in the water-absorbing resin composition of the present invention is composed of a crosslinked polymer obtained by crosslinking a polymer of a water-soluble ethylenically unsaturated monomer, that is, a crosslinked polymer having a structural unit derived from a water-soluble ethylenically unsaturated monomer.

The water-absorbing resin is usually in particulate form. The particulate water-absorbing resin has a median particle diameter of preferably 100 to 600 $\mu$m, more preferably 200 to 500 $\mu$m, and even more preferably 250 to 450 $\mu$m.

Other than a form in which each of the particulate water-absorbing resins is composed of a single particle, the particulate water-absorbing resin may be in a form (secondary particles) in which fine particles (primary particles) are aggregated. Examples of the shape of the primary particle include a substantially spherical shape, an indefinite crushed shape, and a plate shape. Examples of the primary particles produced by reversed-phase suspension polymerization include substantially spherical single particles having a smooth surface shape such as a perfect spherical shape or an elliptical spherical shape. Since the primary particles having such a shape have a smooth surface shape, flowability as a powder becomes high and aggregated particles are easily densely packed. Thus, a water-absorbing resin which is less likely to be broken even when subjected to impact and has high particle strength is obtained.

The median particle diameter of the particulate water-absorbing resin can be measured using JIS standard sieves, and specifically, is a value measured by the method described in EXAMPLES.

As a polymerization method of the water-soluble ethylenically unsaturated monomer, an aqueous solution polymerization method, an emulsion polymerization method, a reversed-phase suspension polymerization method, and the like, which are representative polymerization methods, are used. In the aqueous solution polymerization method, polymerization is performed by heating an aqueous solution of a water-soluble ethylenically unsaturated monomer while stirring the aqueous solution as necessary. In the reversed-phase suspension polymerization method, polymerization is performed by heating a water-soluble ethylenically unsaturated monomer in a hydrocarbon dispersion medium under stirring. A reversed-phase suspension polymerization method is preferably used from the viewpoints of enabling precise control of polymerization reaction and control of wide-ranging particle diameter.

An example of the method for producing the water-absorbing resin will be described below.

Specific examples of the method for producing a water-absorbing resin include a method for producing a water-absorbing resin by performing reversed-phase suspension polymerization of a water-soluble ethylenically unsaturated monomer in a hydrocarbon dispersion medium, the method including a step of performing polymerization in the presence of a radical polymerization initiator and a step of post-crosslinking a hydrous gel obtained by the polymerization in the presence of a post-crosslinking agent. In the method for producing a water-absorbing resin, an internal-crosslinking agent may be added to the water-soluble ethylenically unsaturated monomer as necessary to form a hydrous gel having an internally-crosslinked structure.

<Polymerization Step>

[Water-soluble ethylenically unsaturated monomer]

Examples of the water-soluble ethylenically unsaturated monomer include (meth)acrylic acid (in the present specification, "acrylic" and "methacrylic" are collectively referred to as "(meth)acrylic", the same applies hereinafter) and salts thereof; 2-(meth)acrylamide-2-methylpropanesulfonic acid and salts thereof; nonionic monomers such as (meth)acrylamide, N,N-dimethyl (meth)acrylamide, 2-hydroxyethyl (meth)acrylate, N-methylol (meth)acrylamide, and polyethylene glycol mono(meth)acrylate; amino group-containing unsaturated monomers such as N,N-diethylaminoethyl (meth)acrylate, N,N-diethylaminopropyl (meth)acrylate, and diethylaminopropyl (meth)acrylamide, and quaternized products thereof. Among these water-soluble ethylenically unsaturated monomers, from the viewpoints of industrial availability and the like, (meth)acrylic acid or a salt thereof, (meth)acrylamide, and N,N-dimethylacrylamide are preferable, and (meth)acrylic acid and a salt thereof are more preferable. These water-soluble ethylenically unsaturated monomers may be used singly or in combination of two or more.

Among them, acrylic acid and salts thereof are widely used as raw materials of water-absorbing resins, and these acrylic acid and/or salts thereof may be copolymerized with the above-mentioned other water-soluble ethylenically unsaturated monomers and then used. In this case, acrylic acid and/or a salt thereof is preferably used as a main water-soluble ethylenically unsaturated monomer in an amount of 70 to 100 mol% with respect to the total amount of water-soluble ethylenically unsaturated monomers.

The water-soluble ethylenically unsaturated monomer is preferably dispersed in a state of an aqueous solution in a hydrocarbon dispersion medium and subjected to reversed-phase suspension polymerization. When the water-soluble ethylenically unsaturated monomer is an aqueous solution, the dispersion efficiency in the hydrocarbon dispersion medium can be increased. The concentration of the water-soluble ethylenically unsaturated monomer in this aqueous solution is preferably in a range of 20 mass% to a saturated concentration or less. The concentration of the water-soluble ethylenically unsaturated monomer is more preferably 55 mass% or less, even more preferably 50 mass% or less, and still even more preferably 45 mass% or less. On the other hand, the concentration of the water-soluble ethylenically unsaturated monomer is more preferably 25 mass% or more, even more preferably 28 mass% or more, and still even more preferably 30 mass% or more.

Like (meth)acrylic acid, 2-(meth)acrylamide-2-methylpropanesulfonic acid, or similar monomer, when the water-soluble ethylenically unsaturated monomer has an acid group, the acid group may be neutralized in advance with an alkaline neutralizing agent as necessary prior to use. Examples of such an alkaline neutralizing agent include alkali metal salts such as sodium hydroxide, sodium carbonate, sodium hydrogen carbonate, potassium hydroxide, and potassium carbonate; and ammonia. In addition, these alkaline neutralizing agents may be used in the form of an aqueous solution in order to simplify the neutralization operation. The alkaline neutralizing agents described above may be used singly or in combination of two or more.

The degree of neutralization of the water-soluble ethylenically unsaturated monomer by the alkaline neutralizing agent is preferably 10 to 100 mol%, more preferably 30 to 90 mol%, even more preferably 40 to 85 mol%, and still even more preferably 50 to 80 mol% as the degree of neutralization with respect to all the acid groups of the water-soluble ethylenically unsaturated monomer.

[Radical polymerization initiator]

Examples of the radical polymerization initiator added to the polymerization step include persulfates such as potassium persulfate, ammonium persulfate, and sodium persulfate, peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide, and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethylpropionamide}, 2,2'-azobis[2-methyl-N-(2-hydroxyethyl)-propionamide], and 4,4'-azobis(4-cyanovaleric acid). Among these radical polymerization initiators, potassium persulfate, ammonium persulfate, sodium persulfate, and 2,2'-azobis(2-amidinopropane) dihydrochloride are preferable from the viewpoints of easy availability and easy handling. These radical polymerization initiators may be used singly or in combination of two or more. The radical polymerization initiator can also be used as a redox polymerization initiator in combination with a reducing agent such as sodium sulfite, sodium hydrogen sulfite, ferrous sulfate, or L-ascorbic acid.

The amount of the radical polymerization initiator used is, for example, 0.00005 to 0.01 mol with respect to 1 mol of the water-soluble ethylenically unsaturated monomer. By satisfying such an amount used, occurrence of a rapid polymerization reaction can be avoided, and the polymerization reaction can be completed in an appropriate time.

[Internal-crosslinking agent]

Examples of the internal-crosslinking agent include those capable of crosslinking a polymer of a water-soluble ethylenically unsaturated monomer to be used, and examples thereof include (poly) ethylene glycol ["(poly)" means the case where there is or is not a prefix "poly" the same applies hereinafter], unsaturated polyesters obtained by reacting polyols including diols and triols such as (poly)propylene glycol, 1,4-butanediol, trimethylolpropane, and (poly)glycerin with unsaturated acids such as (meth)acrylic acid, maleic acid, and fumaric acid; bisacrylamides such as N,N-methylenebisacrylamide; di(meth)acrylic acid esters or tri(meth)acrylic acid esters obtained by reacting polyepoxide with (meth)acrylic acid; di(meth)acrylic acid carbamyl esters obtained by reacting polyisocyanates such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds such as diglycidyl compounds including (poly)ethylene glycol diglycidyl ether, (poly)propylene glycol diglycidyl ether, and (poly)glycerin diglycidyl ether and triglycidyl compounds; epihalohydrin compounds such as epichlorohydrin, epibromohydrin, and $\alpha$-methylepichlorohydrin; compounds having two or more reactive functional groups such as isocyanate compounds including 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, unsaturated polyesters and polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are preferably used. These internal-crosslinking agents may be used singly or in combination of two or more.

The amount of the internal-crosslinking agent used is preferably 0.000001 to 0.02 mol, more preferably 0.00001 to 0.01 mol, even more preferably 0.00001 to 0.005 mol, and still even more preferably 0.00005 to 0.002 mol with respect to 1 mol of the water-soluble ethylenically unsaturated monomer.

[Hydrocarbon dispersion medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbon such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly suitably used because they are industrially easily available, have stable quality, and are inexpensive. These hydrocarbon dispersion media may be used singly or in combination of two or more. As an example of the mixture of the hydrocarbon dispersion medium, a suitable result can be obtained by using a commercially available product such as Exxol Heptane (manufactured by Exxon Mobil Corporation: containing 75 to 85 mass% hydrocarbons of heptane and an isomer thereof).

The amount of the hydrocarbon dispersion medium used is preferably 100 to 1500 parts by mass, and more preferably 200 to 1400 parts by mass with respect to 100 parts by mass of the first-stage water-soluble ethylenically unsaturated monomer from the viewpoints of uniformly dispersing the water-soluble ethylenically unsaturated monomer and easily controlling the polymerization temperature. As will be described later, the reversed-phase suspension polymerization is performed in one stage (single stage) or two or more stages, and the above-described first-stage polymerization means the first-stage polymerization reaction in the single-stage polymerization or the multistage polymerization (the same applies hereinafter).

[Dispersion stabilizer]

(Surfactant)

In the reversed-phase suspension polymerization, a dispersion stabilizer can also be used in order to improve the dispersion stability of the water-soluble ethylenically unsaturated monomer in the hydrocarbon dispersion medium. As the dispersion stabilizer, a surfactant can be used.

As the surfactant, for example, a sucrose fatty acid ester, a polyglycerin fatty acid ester, a sorbitan fatty acid ester, a polyoxyethylene sorbitan fatty acid ester, a polyoxyethylene glycerin fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, a phosphoric acid ester of a polyoxyethylene alkyl allyl ether, or the like can be used. Among these surfactants, it is particularly preferable to use a sorbitan fatty acid ester, a polyglycerin fatty acid ester, or a sucrose fatty acid ester from the viewpoint of dispersion stability of the monomer. These surfactants may be used singly or in combination of two or more.

The amount of the surfactant used is preferably 0.1 to 30 parts by mass, and more preferably 0.3 to 20 parts by mass with respect to 100 parts by mass of the first-stage water-soluble ethylenically unsaturated monomer.

(Polymeric dispersant)

As the dispersion stabilizer used in the reversed-phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified ethylene propylene diene methylene linkage (EPDM), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, and ethyl hydroxyethyl cellulose. Among these polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from the viewpoint of dispersion stability of monomers. These polymeric dispersants may be used singly or in combination of two or more.

The amount of the polymeric dispersant used is preferably 0.1 to 30 parts by mass, and more preferably 0.3 to 20 parts by mass with respect to 100 parts by mass of the first-stage water-soluble ethylenically unsaturated monomer.

[Other components]

In the method for producing a water-absorbing resin, if desired, other components may be added to an aqueous solution containing a water-soluble ethylenically unsaturated monomer and then the reversed-phase suspension polymerization may be performed. As other components, various additives such as a thickener and a chain transfer agent can be added.

As an example, reversed-phase suspension polymerization can be performed after adding a thickener to an aqueous solution containing a water-soluble ethylenically unsaturated monomer. By thus adding a thickener to adjust the viscosity of the aqueous solution, it is possible to control the median particle diameter obtained in the reversed-phase suspension polymerization.

As the thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinylpyrrolidone, polyethylene oxide, or the like can be used. When the stirring speed during polymerization is the same, the primary particles and/or the secondary particles of the obtained particles tend to be larger as the viscosity of the aqueous solution of the water-soluble ethylenically unsaturated monomer is higher.

[Reversed-phase suspension polymerization]

In performing the reversed-phase suspension polymerization, for example, an aqueous monomer solution containing a water-soluble ethylenically unsaturated monomer is dispersed in a hydrocarbon dispersion medium in the presence of a dispersion stabilizer. In this case, as long as it is before start of the polymerization reaction, the addition timing of the dispersion stabilizer (surfactant or polymeric dispersant) may be either before or after the addition of the aqueous monomer solution.

Among them, from the viewpoint of easily reducing the amount of the hydrocarbon dispersion medium remaining in the resulting water-absorbing resin, it is preferable to disperse the aqueous monomer solution in the hydrocarbon dispersion medium in which the polymeric dispersant has been dispersed, and then to further disperse the surfactant therein before performing polymerization.

Such reversed-phase suspension polymerization can be performed in one stage or two or more stages. In addition, from the viewpoint of enhancing productivity, it is preferable to perform 2 to 3 stages.

When the reversed-phase suspension polymerization is performed in two or more stages, the first-stage reversed-phase suspension polymerization is performed, then the water-soluble ethylenically unsaturated monomer is added to and mixed with the reaction mixture obtained in the first-stage polymerization reaction, and the second-stage or subsequent reversed-phase suspension polymerization may be performed in the same manner as the first-stage polymerization. In the reversed-phase suspension polymerization in each of the second and subsequent stages, in addition to the water-soluble ethylenically unsaturated monomer, a radical polymerization initiator is preferably added within the range of the molar ratio of each component to the water-soluble ethylenically unsaturated monomer described above based on the amount of the water-soluble ethylenically unsaturated monomer added in the reversed-phase suspension polymerization in each of the second and subsequent stages, to perform reversed-phase suspension polymerization. In the second and subsequent polymerization, an internal-crosslinking agent may be added to the water-soluble ethylenically unsaturated monomer as necessary.

The reaction temperature of the polymerization reaction is preferably 20 to 110°C and more preferably 40 to 90°C from the viewpoints of enhancing the economic efficiency by rapidly proceeding the polymerization and shortening the polymerization time, and smoothly performing the reaction by easily removing the heat of polymerization.

<Post-crosslinking step>

Next, the water-absorbing resin is obtained by adding a post-crosslinking agent to the hydrous gel having an internally-crosslinked structure which has been obtained by polymerizing the water-soluble ethylenically unsaturated monomer, and crosslinking the hydrous gel (post-crosslinking reaction). This post-crosslinking reaction is preferably performed in the presence of a post-crosslinking agent after polymerization of the water-soluble ethylenically unsaturated monomer. As described above, by subjecting the hydrous gel having an internally-crosslinked structure to a post-crosslinking reaction after polymerization, it is possible to obtain a water-absorbing resin in which the crosslinking density in the vicinity of the surface of the water-absorbing resin is increased and various performances such as water absorption capacity under load are improved.

Examples of the post-crosslinking agent include compounds having two or more reactive functional groups. Examples include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compound such as epichlorohydrin, epibromohydrin, and $\alpha$-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compound such as 1,2-ethylene bisoxazoline; carbonate compound such as ethylene carbonate; and hydroxyalkylamide compounds such as bis[N,N-di($\beta$-hydroxyethyl)]adipamide. Among these post-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. These post-crosslinking agents may be used singly or in combination of two or more.

The amount of the post-crosslinking agent used is preferably 0.00001 to 0.01 mol, more preferably 0.00005 to 0.005 mol, and even more preferably 0.0001 to 0.002 mol with respect to 1 mol of the total amount of the water-soluble ethylenically unsaturated monomers used for polymerization.

As a method for adding the post-crosslinking agent, the post-crosslinking agent may be added as it is or as an aqueous solution, or may be added as a solution using a hydrophilic organic solvent as a solvent as necessary. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly or in combination of two or more, or as a mixed solvent obtained by mixing with water.

The addition timing of the post-crosslinking agent may be after almost all the polymerization reaction of the water-soluble ethylenically unsaturated monomer is completed, and the post-crosslinking agent is preferably added in the presence of moisture in the range of 1 to 400 parts by mass, more preferably in the presence of moisture in the range of 5 to 200 parts by mass, even more preferably in the presence of moisture in the range of 10 to 100 parts by mass, and still even more preferably in the presence of moisture in the range of 20 to 60 parts by mass with respect to 100 parts by mass of the water-soluble ethylenically unsaturated monomer. The amount of moisture means the total amount of moisture contained in the reaction system and moisture used as necessary when the post-crosslinking agent is added.

The reaction temperature in the post-crosslinking reaction is preferably 50 to 250°C, more preferably 60 to 180°C, even more preferably 60 to 140°C, and still even more preferably 70 to 120°C. The reaction time of the post-crosslinking reaction is preferably 1 to 300 minutes, and more preferably 5 to 200 minutes.

<Drying step>

The method may include a drying step of removing water, a hydrocarbon dispersion medium, and the like by externally applying energy such as heat and distilling after performing the reversed-phase suspension polymerization described above. When dehydration of the hydrous gel after the reversed-phase suspension polymerization is performed, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. In that case, since the temperature in the system during drying is maintained at an azeotropic temperature with the hydrocarbon dispersion medium or lower, it is preferable from the viewpoint that the resin is less likely to deteriorate. Subsequently, water and the hydrocarbon dispersion medium are distilled off to obtain particles of the water-absorbing resin. Various performances of the water-absorbing resin to be obtained can be controlled by controlling the treatment conditions in the drying step after the polymerization to adjust the amount of water to be removed.

In the drying step, the drying treatment by distillation may be performed under normal pressure or under reduced pressure. From the viewpoint of enhancing the drying efficiency, the drying may be performed under a flow of nitrogen or the like. When the drying treatment is performed under normal pressure, the drying temperature is preferably 70 to 250°C, more preferably 80 to 180°C, even more preferably 80 to 140°C, and still even more preferably 90 to 130°C. When the drying treatment is performed under reduced pressure, the drying temperature is preferably 40 to 160°C, and more preferably 50 to 110°C.

When the post-crosslinking step with the post-crosslinking agent is performed after the polymerization of the monomer is performed by reversed-phase suspension polymerization, the drying step by distillation described above is performed after the completion of the post-crosslinking step. Alternatively, the post-crosslinking step and the drying step may be performed simultaneously.

The water-absorbing resin composition of the present invention may contain an additive according to the purpose, in addition to the supported hydrazide compound. Examples of such additives include inorganic powders, surfactants, oxidizing agents, reducing agents, metal chelating agents, radical chain inhibitors, antioxidants, and antibacterial agents. For example, the flowability of the water-absorbing resin can be improved by adding 0.05 to 5 parts by mass of amorphous silica as an inorganic powder with respect to 100 parts by mass of the water-absorbing resin.

In the water-absorbing resin composition of the present invention, the content of the water-absorbing resin (excluding additives) is preferably 80 mass% or more, more preferably 90 mass% or more, and even more preferably 98 mass% or more.

The water-absorbing resin composition of the present invention is suitably used for suppressing odor caused by acetaldehyde.

2. Absorber, absorbent article

The water-absorbing resin composition of the present invention constitutes an absorber used for, for example, hygienic materials such as sanitary products and disposable diapers, and is suitably used for an absorbent article including the absorber.

Here, the absorber using the water-absorbing resin composition of the present invention contains the water-absorbing resin composition of the present invention. The absorber may further include hydrophilic fibers. Examples of the configuration of the absorber include a sheet-like structure in which a water-absorbing resin is fixed on a nonwoven fabric or between a plurality of nonwoven fabrics, a mixed dispersion obtained by mixing a water-absorbing resin composition and hydrophilic fibers so as to have a uniform composition, a sandwich structure in which a water-absorbing resin is sandwiched between layered hydrophilic fibers, and a structure in which a water-absorbing resin composition and hydrophilic fibers are wrapped with tissue. The absorber may contain other components, for example, an adhesive binder such as a heat-fusible synthetic fiber, a hot melt adhesive, or an adhesive emulsion for enhancing the shape retention of the absorber.

The content of the water-absorbing resin in the absorber is preferably 5 to 100 mass%, more preferably 10 to 95 mass%, even more preferably 20 to 90 mass%, and still even more preferably 30 to 80 mass%.

Examples of the hydrophilic fibers include cellulose fibers such as fluff pulp, mechanical pulp, chemical pulp, and semi-chemical pulp obtained from wood, artificial cellulose fibers such as rayon and acetate, and fibers made of synthetic resins such as hydrophilized polyamide, polyester, and polyolefin. The average fiber length of the hydrophilic fibers is usually 0.1 to 10 mm or may be 0.5 to 5 mm.

The absorber using the water-absorbing resin composition of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side coming in contact with the body, and the liquid-impermeable sheet is disposed on a side opposite to the side coming in contact with the body.

Examples of the liquid-permeable sheet include an air-through type, a spunbond type, a chemical bond type, a needle punch type, and similar type nonwoven fabrics made of fibers such as polyethylene, polypropylene, and polyester, and porous synthetic resin sheets. Examples of the liquid-impermeable sheet include synthetic resin films made of resins such as polyethylene, polypropylene, and polyvinyl chloride. The liquid-permeable sheet is preferably at least one selected from the group consisting of a thermally bonded nonwoven fabric, an air-through nonwoven fabric, a spunbond nonwoven fabric, and a spunbond/melt-blown/spunbond nonwoven fabric.

The basis weight of the liquid-permeable sheet is preferably 5 g/m$^2$ or more and 100 g/m$^2$ or less, and more preferably 10 g/m$^2$ or more and 60 g/m$^2$ or less. The surface of the liquid-permeable sheet may be embossed or perforated in order to improve the liquid diffusibility. The embossing and the perforating can be performed by known methods.

Examples of the liquid-impermeable sheet include a sheet made of a synthetic resin such as polyethylene, polypropylene, or polyvinyl chloride, a sheet made of a nonwoven fabric such as spunbond/melt-blown/spunbond (SMS) nonwoven fabric in which a water-resistant melt-blown nonwoven fabric is sandwiched between high-strength spunbond nonwoven fabrics, and a sheet made of a composite material of these synthetic resins and a nonwoven fabric (for example, a spunbond nonwoven fabric or a spunlace nonwoven fabric). As the liquid-impermeable sheet, a sheet made of a synthetic resin mainly containing a low-density polyethylene (LDPE) resin can also be used. The liquid-impermeable sheet may be, for example, a sheet made of a synthetic resin having a basis weight of 10 to 50 g/m$^2$.

EXAMPLES

Hereinafter, the present invention will be described in detail with reference to an example and comparative examples. However, the present invention is not limited to the example.

The water-absorbing resin compositions obtained in the following example and comparative examples were evaluated in the following various tests. Hereinafter, each evaluation test method will be described.

<Median particle diameter>

The measurement was performed in an environment at a temperature of 25 ± 2°C and a humidity of 50 ± 10%. JIS standard sieves were combined in the following order from the top: a sieve with an opening of 850 μm, a sieve with an opening of 600 μm, a sieve with an opening of 500 μm, a sieve with an opening of 425 μm, a sieve with an opening of 300 μm, a sieve with an opening of 250 μm, a sieve with an opening of 150 μm, and a receptacle.

The water-absorbing resin composition, 50 g, was placed on the uppermost sieve of the combined sieves, and shaken for 10 minutes using a rotating and tapping shaker to perform classification. After classification, the mass of the water-absorbing resin composition retained on each sieve was calculated as a mass percentage with respect to the total amount, and the particle size distribution was determined. With respect to this particle size distribution, by cumulation of the mass percentages of the retained on the sieves in descending order of particle diameter, the relationship between the opening of the sieve and the cumulative value of the mass percentage of the water-absorbing resin retained on the sieve was plotted on a logarithmic probability paper. The plotted points on the probability paper were connected with a straight line, and a particle diameter corresponding to a cumulative mass percentage of 50 mass% was defined as a median particle diameter.

<Yellow index of water-absorbing resin composition>

In an environment of a temperature of 25 ± 2°C and a humidity of 50 ± 10%, 2.0 g of the water-absorbing resin composition was placed in a glass measuring container (cylindrical, inner diameter 3 cm). The yellow index of the water-absorbing resin composition was measured with a color difference meter (Color Meter ZE6000, manufactured by Nippon Denshoku Industries Co., Ltd.) in which X, Y, and Z, which are tristimulus values of a colorimetric color difference meter, had been corrected, using a white plate for standard use. The yellow index was calculated from X, Y, and Z (tristimulus values) of the obtained water-absorbing resin composition according to the following equation, and defined as an initial yellow index.

$$\text{Yellow index} = 100 \times (1.2769 \times X - 1.0592 \times Z)/Y$$

The test (accelerated test) of coloring of the water-absorbing resin composition over time was performed according to the following procedure. First, 2.0 g of a sample was uniformly placed in a glass container (cylindrical, inner diameter 3 cm, depth 1 cm), and the container was stored for 7 days in a tabletop thermo-hygrostat set at a temperature of 70 ± 2°C and a relative humidity of 90 ± 2%. After a lapse of 7 days, the container was taken out from the thermo-hygrostat, and left for a while to be cooled to room temperature. The yellow index of the water-absorbing resin composition was measured with a color difference meter (Color Meter ZE6000, manufactured by Nippon Denshoku Industries Co., Ltd.). From X, Y, and Z (tristimulus values) of the obtained water-absorbing resin composition, the yellow index of the water-absorbing resin composition was calculated according to the following equation. The yellow index is shown in Table 1.

$$\text{Yellow index} = 100 \times (1.2769 \times X - 1.0592 \times Z)/Y$$

<Acetaldehyde odor suppression test>

The acetaldehyde odor suppression test was performed in an environment of 25°C $\pm$ 2°C. In a plastic petri dish having an inner diameter of 10 cm and a height of 1.5 cm, 10.00 g of the water-absorbing resin composition was placed. The petri dish containing the water-absorbing resin composition was placed in a 2L-Tedlar bag (with one port and a cap) equipped with a three-way cock with a silicone tube having an inner diameter of 5 mm interposed therebetween, and the bag was hermetically sealed by heat sealing. Then, the glass syringe (constant humidity glass syringe, 200 mL, manufactured by Tsuji Seisakusho Co., Ltd.) was connected to a three-way cock, and the entire amount of air in the Tedlar bag was withdrawn. Thereafter, 900 mL of a standard gas having an acetaldehyde concentration of 20 $\pm$ 1 ppm (manufactured by Sumitomo Seika Chemicals Co., Ltd., nitrogen dilution, analytical value 19.3 ppm) was enclosed in the bag using the above-described glass syringe. After 60 minutes from the completion of enclosing of the acetaldehyde-containing gas, the three-way cock was removed from the Tedlar bag, a gas detection tube with the ends open (manufactured by GASTEC Corporation, detection tube: acetaldehyde 92L) was attached, and the acetaldehyde concentration in the Tedlar bag gas phase was measured. The measurement results are shown in Table 1.

<Production of water-absorbing resin>

(Production Example 1)

A 2-L round-bottom cylindrical separable flask having an inner diameter of 11 cm and equipped with a reflux condenser, a dropping funnel, a nitrogen gas inlet tube, and a stirring blade having two stages of 4-inclined paddle blades having a blade diameter of 5 cm as a stirrer was prepared. To this flask, 293 g of n-heptane as a hydrocarbon dispersion medium and 0.736 g of a maleic anhydride-modified ethylene-propylene copolymer (Mitsui Chemicals, Inc., Hi-WAX 1105A) as a polymeric dispersant were added, the temperature was raised to 80°C with stirring to dissolve the dispersant, and then the contents were cooled to 50°C.

On the other hand, in a beaker having an internal volume of 300 mL, 92.0 g (1.03 mol) of an 80.5 mass% aqueous acrylic acid solution as a water-soluble ethylenically unsaturated monomer was placed, 147.7 g of a 20.9 mass% aqueous sodium hydroxide solution was added dropwise while cooling with ice water to perform neutralization of 75 mol%, and then 0.092 g (Sumitomo Seika Chemicals Co., Ltd., HECAW-15F) of hydroxylethyl cellulose as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added thereto and dissolved, thereby preparing a first-stage aqueous monomer solution.

Then, the aqueous monomer solution prepared above was added to the separable flask and stirred for 10 minutes, after which a surfactant solution obtained by heating and dissolving 0.736 g of a sucrose stearate having an HLB of 3 (Mitsubishi Chemical Foods Corporation, Ryoto Sugar Ester S-370) as a surfactant in 6.62 g of n-heptane in a 20 mL-vial was further added. While stirring at a stirrer rotational speed of 550 rpm, the inside of the system was sufficiently purged with nitrogen, and then the flask was immersed in a water bath at 70°C for 60 minutes to obtain a first-stage polymerization slurry solution.

On the other hand, in another beaker having an internal volume of 500 mL, 128.8 g (1.43 mol) of an 80.5 mass% aqueous acrylic acid solution as a water-soluble ethylenically unsaturated monomer was placed, 159.0 g of a 27 mass% aqueous sodium hydroxide solution was added dropwise while cooling with ice water to perform neutralization of 75 mol%, and then 0.103 g (0.381 mmol) of potassium persulfate as a water-soluble radical polymerization initiator and 0.0116 g (0.067 mmol) of ethylene glycol diglycidyl ether as an internal-crosslinking agent were added thereto and dissolved, thereby preparing a second-stage aqueous monomer solution.

While stirring at a stirrer rotational speed of 1000 rpm, the inside of the separable flask system was cooled to 25°C. Then the whole amount of the second-stage aqueous monomer solution was added to the first-stage polymerization slurry. After the inside of the system was purged with nitrogen for 30 minutes, the flask was immersed again in a water bath at 70°C for 60 minutes to obtain a second-stage hydrous gel polymer.

To the hydrous gel polymer after the second-stage polymerization, 0.589 g of a 45 mass% aqueous pentasodium diethylenetriaminepentaacetate solution was added under stirring. Thereafter, the flask was immersed in an oil bath set at 125°C, and 257.7 g of water was removed to the outside of the system while refluxing n-heptane by azeotropic distillation of n-heptane and water. Then, 4.42 g (0.507 mmol) of a 2 mass% aqueous solution of ethylene glycol diglycidyl ether as a post-crosslinking agent was added to the flask, and the contents were held at 83°C for 2 hours.

Thereafter, the contents were dried by evaporation of n-heptane at 125°C to obtain a particulate crosslinked polymer (dry product). This particulate crosslinked polymer was passed through a sieve with an opening of 850 $\mu$m, and

0.1 mass% of amorphous silica (Oriental Silicas Corporation, TOKUSIL NP-S) with respect to the mass of the particulate crosslinked polymer was mixed with the particulate crosslinked polymer to obtain 228.0 g of a particulate water-absorbing resin containing amorphous silica. The median particle diameter of the particulate water-absorbing resin was 352 $\mu$m.

<Production of water-absorbing resin composition>

(Example 1)

To 100 parts by mass of the water-absorbing resin obtained in Production Example 1, 0.100 parts by mass of a silicate compound on which a hydrazide compound was supported (Sinanen Zeomic Co., Ltd., Dushlite M, supported amount of hydrazide compound; 7 mass%, median particle diameter 6 $\mu$m) was powder-mixed to obtain a water-absorbing resin composition. The yellow index of this water-absorbing resin composition on the 7th day of the accelerated test was 9.5, and the acetaldehyde concentration in the gas phase after the acetaldehyde odor suppression test was 3 ppm.

(Comparative Example 1)

To 100 parts by mass of the water-absorbing resin obtained in Production Example 1, an aqueous malonic acid dihydrazide solution containing 0.007 parts by mass of malonic acid dihydrazide and 0.133 parts by mass of deionized water was added dropwise under stirring of the water-absorbing resin, and then the mixture was heated at 105°C for 30 minutes. Then, the resultant was passed through a JIS standard sieve having an opening of 850 $\mu$m to obtain a water-absorbing resin composition. The yellow index of this water-absorbing resin composition on the 7th day of the accelerated test was 10.4, and the acetaldehyde concentration in the gas phase after the acetaldehyde odor suppression test was 18 ppm.

(Comparative Example 2)

To 100 parts by mass of the water-absorbing resin obtained in Production Example 1, an aqueous malonic acid dihydrazide solution containing 0.035 parts by mass of malonic acid dihydrazide and 0.665 parts by mass of deionized water was added dropwise under stirring of the water-absorbing resin, and then the mixture was heated at 105°C for 30 minutes. Then, the resultant was passed through a JIS standard sieve having an opening of 850 $\mu$m to obtain a water-absorbing resin composition. The yellow index of this water-absorbing resin composition on the 7th day of the accelerated test was 14.6, and the acetaldehyde concentration in the gas phase after the acetaldehyde odor suppression test was 18 ppm.

(Comparative Example 3)

To 100 parts by mass of the water-absorbing resin obtained in Production Example 1, an aqueous malonic acid dihydrazide solution containing 0.070 parts by mass of malonic acid dihydrazide and 1.33 parts by mass of deionized water was added dropwise under stirring of the water-absorbing resin, and then the mixture was heated at 105°C for 30 minutes. Then, the resultant was passed through a JIS standard sieve having an opening of 850 $\mu$m to obtain a water-absorbing resin composition. The yellow index of this water-absorbing resin composition on the 7th day of the accelerated test was 16.5, and the acetaldehyde concentration in the gas phase after the acetaldehyde odor suppression test was 18 ppm.

(Comparative Example 4)

To 100 parts by mass of the water-absorbing resin obtained in Production Example 1, 0.093 parts by mass of silicate (corresponding to the support used in Example 1, median particle diameter 6 $\mu$m) was powder-mixed to obtain a water-absorbing resin composition. The yellow index of this water-absorbing resin composition on the 7th day of the accelerated test was 20.6, and the acetaldehyde concentration in the gas phase after the acetaldehyde odor suppression test was 17 ppm.

(Comparative Example 5)

The water-absorbing resin obtained in Production Example 1 was used as it was as a water-absorbing resin of Comparative Example 5. The yellow index of this water-absorbing resin on the 7th day of the accelerated test was 9.8, and the acetaldehyde concentration in the gas phase after the acetaldehyde odor suppression test was 19 ppm.

[Table 1]

| | Supported hydrazide compound | | Yellow index of water-absorbing resin | | Acetaldehyde concentration after odor suppression test |
|---|---|---|---|---|---|
| | Type | Addition amount (parts by mass) | Day 0 | Day 7 | (ppm) |
| Example 1 | Silicate supported malonic acid dihydrazide | 0.100 | 6.2 | 9.5 | 3 |
| Comparative example 1 | Malonic acid dihydrazide | 0.007 | 6.4 | 10.4 | 18 |
| Comparative example 2 | Malonic acid dihydrazide | 0.035 | 7.7 | 14.6 | 18 |
| Comparative example 3 | Malonic acid dihydrazide | 0.070 | 7.4 | 16.5 | 18 |
| Comparative example 4 | Silicate | 0.093 | 6.7 | 20.6 | 17 |
| Comparative example 5 | - | | 6.2 | 9.8 | 19 |

**Claims**

1. A water-absorbing resin composition comprising:

    a supported hydrazide compound; and
    a water-absorbing resin,
    wherein
    the water-absorbing resin composition has a yellow index of 10 or less when 2.0 g of the water-absorbing resin composition is held in an environment at a temperature of 70°C and a relative humidity of 90% for 7 days, and an acetaldehyde concentration of 15 ppm or less is measured when the water-absorbing resin composition is tested according to the acetaldehyde odor suppression test as disclosed in the description.

2. The water-absorbing resin composition according to claim 1, wherein

    the water-absorbing resin composition is in particulate form, and
    the supported hydrazide compound is present on at least one of a surface and an inside of the water-absorbing resin composition.

3. The water-absorbing resin composition according to claim 1 or 2, wherein a support of the supported hydrazide compound is at least one of silicic acid and a silicate.

4. The water-absorbing resin composition according to any one of claims 1 to 3, wherein a content of the supported hydrazide compound is 0.001 to 10 mass%.

5. The water-absorbing resin composition according to any one of claims 1 to 4, wherein a content of a hydrazide compound in the supported hydrazide compound is 0.1 to 30 mass%.

6. The water-absorbing resin composition according to any one of claims 1 to 5, wherein the supported hydrazide compound has a median particle diameter of 0.01 to 100 $\mu$m.

7. An absorber comprising the water-absorbing resin composition according to any one of claims 1 to 6.

8. An absorbent article comprising the absorber according to claim 7 held between a liquid-permeable sheet and a liquid-impermeable sheet.

**Patentansprüche**

1. Wasser absorbierende Harzzusammensetzung, die Folgendes umfasst:

   eine Hydrazidverbindung auf einem Träger; und
   ein Wasser absorbierendes Harz,
   wobei
   die Wasser absorbierende Harzzusammensetzung einen Gelbindex von 10 oder weniger aufweist, wenn 2,0 g der Wasser absorbierenden Harzzusammensetzung 7 Tage lang in einer Umgebung mit einer Temperatur von 70 °C und einer relativen Feuchte von 90 % gehalten werden, und
   eine Acetaldehyd-Konzentration von 15 ppm oder weniger gemessen wird, wenn die Wasser absorbierende Harzzusammensetzung gemäß dem in der Beschreibung offenbarten Acetaldehydgeruch-Unterdrückungstest getestet wird.

2. Wasser absorbierende Harzzusammensetzung nach Anspruch 1, wobei

   die Wasser absorbierende Harzzusammensetzung in partikulärer Form vorliegt und
   die Hydrazidverbindung auf einem Träger auf zumindest einem aus einer Oberfläche und dem Inneren der Wasser absorbierenden Harzzusammensetzung vorhanden ist.

3. Wasser absorbierende Harzzusammensetzung nach Anspruch 1 oder 2, wobei ein Träger der Hydrazidverbindung auf einem Träger zumindest eines aus Kieselsäure und einem Silicat ist.

4. Wasser absorbierende Harzzusammensetzung nach einem der Ansprüche 1 bis 3, wobei der Gehalt der Hydrazidverbindung auf einem Träger 0,001 bis 10 Masse-% beträgt.

5. Wasser absorbierende Harzzusammensetzung nach einem der Ansprüche 1 bis 4, wobei der Gehalt der Hydrazidverbindung in der Hydrazidverbindung auf einem Träger 0,1 bis 30 Masse-% beträgt.

6. Wasser absorbierende Harzzusammensetzung nach einem der Ansprüche 1 bis 5, wobei die Hydrazidverbindung auf einem Träger einen mittleren Teilchendurchmesser von 0,01 bis 100 $\mu$m aufweist.

7. Absorber, der eine Wasser absorbierende Harzzusammensetzung nach einem der Ansprüche 1 bis 6 umfasst.

8. Absorptionsgegenstand, der einen Absorber nach Anspruch 7 umfasst, der zwischen einer flüssigkeitsdurchlässigen Lage und einer flüssigkeitsundurchlässigen Lage gehalten wird.

**Revendications**

1. Composition de résine absorbant l'eau, comprenant :

   un composé d'hydrazide supporté ; et
   une résine absorbant l'eau,
   dans laquelle
   la composition de résine absorbant l'eau présente un indice de jaune de 10 ou moins lorsque 2,0 g de la composition de résine absorbant l'eau sont maintenus dans un environnement à une température de 70 °C et une humidité relative de 90 % pendant 7 jours, et
   une concentration d'acétaldéhyde de 15 ppm ou inférieure est mesurée lorsque la composition de résine absorbant l'eau est testée selon le test de suppression des odeurs d'acétaldéhyde tel que divulgué dans la description.

2. Composition de résine absorbant l'eau selon la revendication 1, dans laquelle

   la composition de résine absorbant l'eau est sous forme particulaire, et

le composé d'hydrazide supporté est présent sur au moins un parmi une surface et un intérieur de la composition de résine absorbant l'eau.

3. Composition de résine absorbant l'eau selon la revendication 1 ou 2, dans laquelle un support du composé d'hydrazide supporté est au moins l'un d'un acide silicique et d'un silicate.

4. Composition de résine absorbant l'eau selon l'une quelconque des revendications 1 à 3, dans laquelle une teneur du composé d'hydrazide supporté est de 0,001 à 10 % en masse.

5. Composition de résine absorbant l'eau selon l'une quelconque des revendications 1 à 4, dans laquelle une teneur d'un composé d'hydrazide dans le composé d'hydrazide supporté est de 0,1 à 30 % en masse.

6. Composition de résine absorbant l'eau selon l'une quelconque des revendications 1 à 5, dans laquelle le composé d'hydrazide supporté présente un diamètre de particule médian de 0,01 à 100 $\mu$m.

7. Absorbeur comprenant la composition de résine absorbant l'eau selon l'une quelconque des revendications 1 à 6.

8. Article absorbant comprenant l'absorbeur selon la revendication 7 maintenu entre une feuille perméable aux liquides et une feuille imperméable aux liquides.

**EP 4 144 803 B1**

**REFERENCES CITED IN THE DESCRIPTION**

**Patent documents cited in the description**

- JP 2001323155 A
- JP 2007054328 A
- US 20180221528 A1
- EP 3604366 A1
- JP 2018069140 A
- JP 2017197884 A
- US 20110068300 A1
- JP 2017176757 A
- WO 2007074816 A1
- US 20200030482 A1
- JP 2020050751 A